(19)

Europäisches
Patentamt
European
Patent Office
Office européen
des brevets

(11) **EP 1 523 998 B1**

(12) **EUROPEAN PATENT SPECIFICATION**

(45) Date of publication and mention
of the grant of the patent:
**12.10.2011 Bulletin 2011/41**

(51) Int Cl.:
*A61L 15/46* $^{(2006.01)}$    *A61L 15/58* $^{(2006.01)}$

(21) Application number: **04024214.1**

(22) Date of filing: **11.10.2004**

(54) **Antimicrobial adhesive sheet**

Antimikrobielle haftende Schicht

Feuille adhésive antimicrobienne

(84) Designated Contracting States:
**AT BE BG CH CY CZ DE DK EE ES FI FR GB GR
HU IE IT LI LU MC NL PL PT RO SE SI SK TR**

(30) Priority: **16.10.2003 JP 2003355892**

(43) Date of publication of application:
**20.04.2005 Bulletin 2005/16**

(73) Proprietor: **NITTO DENKO CORPORATION
Osaka (JP)**

(72) Inventors:
• **Hamada, Atsushi
Ibaraki-shi
Osaka (JP)**
• **Okada, Katsuhiro
Ibaraki-shi
Osaka (JP)**

• **Kinoshita, Takashi
Ibaraki-shi
Osaka (JP)**
• **Nakanishi, Tokuji
Ibaraki-shi
Osaka (JP)**
• **Sawada, Yukisama
Hirosaki-shi
Aomori (JP)**

(74) Representative: **Grünecker, Kinkeldey,
Stockmair & Schwanhäusser
Anwaltssozietät
Leopoldstrasse 4
80802 München (DE)**

(56) References cited:
**WO-A-00/74737    US-A- 4 675 395**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 523 998 B1

**Description**

FIELD OF THE INVENTION

The present invention relates to an antimicrobial adhesive sheet and an antimicrobial method using the same. More specifically, the invention relates to an antimicrobial adhesive sheet exerting an antimicrobial activity not only on the surface of an attached site thereof but also in the space in the vicinity of the attached site thereof, by using a specific support. The invention also relates to an antimicrobial method.

BACKGROUND OF THE INVENTION

As medical sheets for attachment on the skin surface, various types have been developed so far, including for example adhesive dressings, winding-type adhesive tapes, first-aid adhesive tape, adhesive bandages, cataplasm plasters containing pharmaceutical agents for topical use, transdermal tape preparations containing pharmaceutical agents for systemic use. An example of antimicrobial adhesive sheet is disclosed in WO-00/74737.

When these medical attachment sheets are attached on injured skin for curing the skin, the injured site may sometimes be contaminated with bacteria existing on skin surface or invading bacteria from the outside after the attachment, which sometimes work to delay the curing even if the medical sheets are preliminarily sterilized during the production and then sealed and packaged. It is remarked that such medical sheets may sometimes cause nosocomial infection. However, such problem is not so important when these medical sheets are attached on normal skin.

In such case, therefore, skin surface is disinfected before attachment or medical attachment sheets have been developed, which contain antimicrobial agents in the adhesive layer thereof (See Reference 1).

Antimicrobial agents with wide antimicrobial spectra have been generally used as the antimicrobial agents for this use. For example, antibiotics such as erythromycin, tetracycline, chloramphenicol, fradiomycin, and streptomycin, compounds with surface activity such as biguanide compounds and benzalkonium, phenol compounds such as phenol and resorcin, iodine compounds such as iodine and povidone iodine, metals such as silver and copper, antimicrobial dye compounds such as acrinol and methylrozanilinium, and sulfur agents such as sulfadiazine and sulfisomidin are used.

When those antimicrobial agents are used in the form of a medical sheet for attachment on skin, however, antimicrobial agents from natural origins when used tend to be less irritating than synthetic compounds. In recent years, hinokitiol contained as a naturally occurring antimicrobial agent in the essential oils of Taiwan Chamaecyparis obtuba and Thujopsis dolabrata SIEBOLD et ZUCCHARINI var. hondae Makino has been drawing attention as having a wide antimicrobial spectrum and a high antimicrobial activity.

As such hinokitiol-containing medical sheets for attachment, an adhesive sheet for covering injuries has been proposed, where hinokitiol as a disinfectant is blended in an acrylic adhesive layer containing zinc oxide (See Reference 2).

The astringent, preservative and protective actions of zinc oxide are expected for the adhesive sheet for covering injuries, as an improved product of the zinc oxide ointment including zinc oxide and lanolin in the related art. Disinfectants such as hinokitiol are contained therein so as to skip the sterilization process during the production process. Thus, a positive antimicrobial activity of hinokitiol is not expected.

Additionally, hinokitiol causes much trouble in handling because hinokitiol is readily vaporizable during heating process, with respect to production of an adhesive sheet containing a vaporizable antimicrobial agent such as hinokitiol. It has not been examined to make an antimicrobial atmosphere by allowing the vaporizable antimicrobial agent to exert the high antimicrobial activity and wide antimicrobial spectrum not only at an attachment site but also even in the space in the vicinity of the attached site, using its readily vaporizable property effectively.

Reference 1: JP-A-61-2862
Reference 2: JP-A-2002-272831

SUMMARY OF THE INVENTION

In accordance with the present invention, antimicrobial adhesive sheets containing antimicrobial agents among medical attachment sheets are improved. It is an object of the present invention to provide an antimicrobial adhesive sheet capable of securely showing antimicrobial effect at an attached site thereof, particularly at a site to be readily bacterially infected, such as injured skin, by allowing the antimicrobial activity to be exerted not only at the attached site but also in the space in the vicinity of the attached site.

After extensive investigations for attaining the above object, it was found that an antimicrobial adhesive sheet capable of exerting an excellent antimicrobial activity could be obtained by allowing a non-aqueous adhesive layer which contains substantially no water to contain a vaporizable antimicrobial agent typically including hinokitiol, using a specific support with a specific property. Thus, the present invention has been achieved.

Thus, the antimicrobial adhesive sheet of the present invention comprises a support and a non-aqueous adhesive layer provided on one side of the support, said adhesive layer comprising an adhesive and a vaporizable antimicrobial agent and the antimicrobial adhesive sheet having a moisture permeability of at least 1,000 g/m2 24 hrs as a whole sheet, on an adherend allowing the antimicrobial adhesive sheet to exert the antimicrobial activity on the surface of the non-aqueous adhesive layer in contact to the adherend and simultaneously vaporizing the vaporizable antimicrobial agent from the back side of the support so as to make the space in the vicinity of the attached site into

Furthermore, the antimicrobial method of the present invention comprises attaching the antimicrobial adhesive sheet of the invention on an adherend allowing the antimicrobial adhesive sheet to exert the antimicrobial activity on the surface of the non-aqueous adhesive layer in contact to the adhered and simultaneously vaporizing the vaporizable antimicrobial agent from the back side of the support so as to make the space in the vicinity of the attached site into an antimicrobial atmosphere.

The antimicrobial adhesive sheet using a support of specific moisture permeability in accordance with the invention can exert the antimicrobial effect on an attached site, for example injured site and can also turn the space in the vicinity of the attached site into an antimicrobial atmosphere because the antimicrobial adhesive sheet can vaporize the vaporizable pharmaceutical agent actively from the back side of the support, since the non-aqueous adhesive containing substantially no water is used in the adhesive layer containing the vaporizable pharmaceutical agent such as hinokitiol. Accordingly, the antimicrobial adhesive sheet can exert an effect of exerting an excellent antimicrobial activity as a whole. Therefore, the antimicrobial sheet can effectively prevent bacterial infection such as nosocomial infection in a secure manner even when the antimicrobial sheet is attached on injured skin.

## DETAILED DESCRIPTION OF THE INVENTION

As the support for use in the antimicrobial adhesive sheet of the present invention, a support that has specific moisture permeability while having an adhesive layer formed on one side thereof should be selected. That is, in accordance with the present invention, a support having a moisture permeability of at least 1,000 $g/m^2$ • 24 hrs as the whole sheet should be used so as to vaporize a vaporizable antimicrobial agent contained in the non-aqueous adhesive layer into the atmosphere from the side opposite to the side with the adhesive layer formed thereon. By using a support having a moisture permeability of the above value, the antimicrobial agent vaporizes from the back side of the support when the antimicrobial adhesive sheet of the present invention is attached on an attached site, so that the space in the vicinity of the attached site can be made into an antimicrobial atmosphere. Herein, the moisture permeability of the adhesive layer itself has very high moisture permeability compared with the moisture permeability of the support, so that it can be neglected. Thus, substantially, the moisture permeability of the whole sheet can approximately be determined on the basis of the moisture permeability of the support.

As the support with such high moisture permeability, for example, non-woven fabric and porous plastic sheet can be used. Examples of the non-woven fabric include non-woven fabrics made of various plastic materials such as polyolefinic resins such as polyethylene and polypropylene, polyester resins such as polyethylene terephthalate, and rayon, nylon, vinylon and cuprammonium rayon, as well as non-woven pulp fabric. From the standpoints of handing during the production and the attachment procedure of the antimicrobial adhesive sheet and the uncomfortable feeling during attachment, the non-woven fabric for use preferably has a weight of 5 to 100 $g/m^2$ • 24hrs, more preferably 25 to 45 $g/m^2$ • 24hrs. When the weight is too small, the non-woven fabric essentially has a poor self-supporting property so that the handing is deteriorated. When the weight is too large, the non-woven fabric would have poor softness, so that the fabric may give stiff feeling (uncomfortable feeling) to the attached skin surface.

The porous plastic sheet specifically includes sheets made of plastic materials, for example, polyolefin resins such as polyethylene, polypropylene, ethylene/vinyl acetate copolymer, polyethylene/ethyl acrylate copolymer, and polytetrafluoroethylene; polyester resins such as polyethylene terephthalate; polyurethane resins such as polyether polyurethane and polyester polyurethane; polyamide resins such as nylon and polyether polyamide block copolymer; and polyvinyl chloride, Saran, and Surlyn. The means for preparing porous material includes, for example, perforation treatment for non-porous plastic sheet, known treatments for preparing porous materials during a process of producing a sheet, for example, a treatment including a step of blending inorganic powders or organic solvents in a material to be prepared into sheet, to thereby make the resulting mixture into sheet, a step of drawing of the sheet when necessary, and a final step of extracting the organic solvents or the inorganic powders to prepare the resulting sheet into a porous material. In case of such non-woven fabric and porous plastic sheet, the moisture permeability thereof is high so that the vaporization property of the vaporizable antimicrobial agent becomes very high. Such fabric or sheet is effective, for example, in case of attachment on a site with no exposure to atmospheric air or on normal skin so as to turn only the space in the vicinity of the attached site into antimicrobial environment.

As the support of the present invention, additionally, non-porous plastic sheet other than the non-woven fabric and porous plastic sheet described above may also be used. As the materials composing such plastic sheet, the same materials as for the porous plastic sheet can be used. In case of non-porous plastic sheet, it should have a moisture permeability of 1,000 to 4,000 g/m$^2$ • 24hrs, preferably 1,000 to 3,000 g/m$^2$ • 24hrs, and more preferably 1,000 to 2,000 g/m$^2$ • 24hrs, so as to allow the non-porous plastic sheet to exert the advantage of the present invention, namely the release of the vaporizable antimicrobial agent from the back side of the support. In other words, though the non-porous plastic sheet has a small moisture permeability level than that of the porous plastic sheet, it is sufficient for the non-porous plastic sheet to secure a moisture permeability of at least 1,000 to 4,000 g/m$^2$ • 24hrs for the exertion of the advantages of the present invention. As a non-porous plastic sheet with such moisture permeability, further, polyurethane resins such as polyether polyurethane and polyamide resins such as polyether polyamide block copolymer are preferably used, owing not only to the moisture permeability but also to the softness thereof, so that no uncomfortable feeling occurs even when the sheet is attached on skin. In case that the non-porous plastic sheet or the porous plastic sheet is used in the support, the thickness thereof is preferably about 10 to 100 μm, more preferably about 20 to 50 μm, so as to avoid the occurrence of the uncomfortable feeling when the sheet is attached on a skin face, particularly on a site with a larger skin motion, such as joint part. In order for the sheet to follow the skin motion at the attached skin face, preferably, the tensile strength is adjusted to about 100 to 900 kg/cm$^2$ and the 100 % modulus is adjusted to about 10 to 100 kg/cm$^2$.

When such non-porous plastic sheet is used as the support, the vaporization of the vaporizable antimicrobial agent contained in the adhesive layer can be sustained. Thus, such non-porous plastic sheet is effective for the attachment on a site exposed to atmospheric air or for a case that the sheet should be attached for a period as long as one week or longer.

Depending on the purpose, a support made of the non-woven fabric or the porous plastic sheet or a support made of the non-porous plastic sheet can be used as the support for use in accordance with the present invention. In order to exert the antimicrobial effect of the vaporizable antimicrobial agent contained in the adhesive layer, namely the antimicrobial activity for the attached site and the antimicrobial activity for the atmosphere in the vicinity of the attached site in a sustained manner, the non-porous plastic sheet is effectively used. The control of the sustainability may be achieved in this case by selecting a plastic sheet material or by lamination of the non-woven fabric, the porous plastic sheet and the non-porous plastic sheet. In a more simple manner, the control thereof can be achieved by laminating an underlining sheet having a moisture permeability of 500 g/m$^2$ • 24hrs or less, preferably 200 g/m$^2$ • 24hrs or less, more preferably substantially no moisture permeability in a removable manner on the back side of the support from which the vaporizable antimicrobial agent vaporizes.

Because the underlining sheet has a moisture permeability smaller than that of the support, the vaporization of the vaporizable antimicrobial agent is suppressed during the lamination of the underlining sheet. By removing the sheet when necessary, the vaporizability is raised to enhance the antimicrobial activity in the atmosphere in the vicinity of the attached site, so that the vaporizable antimicrobial agent contained in the adhesive layer can efficiently be utilized in a sustained manner.

As the underlining sheet with such moisture permeability, various plastic sheets, plastic sheets vapor-deposited with metal foil and metal, laminate sheets of plastic sheet and metal foil, paper base laminated with plastic sheet and the like can be used. The control of the moisture permeability may satisfactorily be achieved by the material and the sheet thickness. Additionally, the means for laminating the underlining sheet on the back side of the support in a removable manner includes a method of interposing an adhesive and a binder resin. When thermo-melting underlining sheets such as thermoplastic plastic sheet are used, otherwise, such underlining sheets can be laminated in a simple manner by contact bonding under heating.

The adhesive layer in accordance with the present invention is formed on one side of the support, contains and retains a vaporizable antimicrobial agent and is formed with a non-aqueous adhesive containing substantially no water. Herein, the term "substantially no water" means that no water is contained during the production process. Therefore, it does not means that the adhesive layer never contains any moisture spontaneously absorbed when the adhesive layer is exposed to air. When water exists in the adhesive layer in the present invention, water may potentially promote the vaporizable antimicrobial agent contained gets to become unstable via heat or light. Particularly in the case that the vaporizable antimicrobial agent is included in cyclodextrin or is in a salt form, the vaporizable antimicrobial agent may readily turn its free form due to the existence of water in the adhesive layer, so that there may be fear that effects of the present invention potentially cannot be exerted sufficiently during use.

As the non-aqueous adhesive which forms such adhesive layer, adhesives such as acrylic adhesives, natural rubber-based adhesives, synthetic rubber-based adhesives, silicone adhesives, vinyl ether adhesives and polyester adhesives may be used. Among them, acrylic adhesives are preferably used in view of the stability of adhesive quality, the easy control of the adhesive property, the long-term stability of the adhesive property and no irritation on skin. As the acrylic adhesives, a copolymer prepared by copolymerizing (meth)acrylic acid alkyl ester as the main component monomer with a monomer copolymerizable with the monomer is preferably used.

Illustrative examples of the (meth)acrylic acid alkyl ester include acrylic acid and methacrylic acid bound through ester bonding with an alcohol with 1 to 18 carbon atoms, preferably 4 to 12 carbon atoms may be used. Preferable specific examples thereof include butyl (meth)acrylate, pentyl (meth)acrylate, hexyl (meth)acrylate, heptyl (meth)acrylate, octyl (meth)acrylate, nonyl (meth)acrylate, decyl(meth)acrylate and dodecyl(meth)acrylate. Among them, (meth)acrylic acid alkyl esters with a long chain alkyl ester with an alkyl group with 6 or more carbon atoms, particularly 6 to 18 carbon atoms are preferably used. Herein, the alkyl ester chain of the (meth)acrylic acid alkyl ester in accordance with the present invention includes alkyl ester chains in not only linear chains but also branched chains as structural isomers.

The (meth)acrylic acid alkyl ester may be prepared in the form of a homopolymer made of single type or in the form of a copolymer of a combination of such two types or more as an adhesive. In order to readily adjust adhesive properties such as skin adhesion, inner aggregation property and tack (adhesiveness), it is preferable to copolymerize copolymerizable monomers. As The copolymerizable monomer, monomers with carboxyl group, sulfonyl group, amino group, amido group, vinyl ester group, alkoxyl group, hydroxyl group and the like within the molecules may be used. Illustrative examples of the monomer includes carboxyl group-containing monomers such as acrylic acid, methacrylic acid, itaconic acid, crotonic acid, maleic acid, fumaric acid, mesaconic acid, citraconic acid, and glutaconic acid; sulfoxyl group-containing monomers such as styrenesulfonic acid, allylsulfonic acid, and sulfopropyl (meth)acrylate; amino group-containing monomers such as aminoethyl (meth)acrylate, dimethylaminoethyl (meth)acrylate, and tert-butylaminoethyl (meth)acrylate; amide group-containing monomers such as acrylamide, methacrylamide, N-vinyl-2-pyrrolidone, dimethyl (meth)acrylamide, and N-butylacrylamide; vinyl ester group-containing monomers such as vinyl acetate and vinyl propionate; alkoxyl group-containing monomers such as methoxypolyethylene glycol (meth)acrylate, ethoxydiethylene glycol (meth)acrylate, butoxydiethylene glycol (meth)acrylate, methoxyethyl (meth)acrylate, and ethoxyethyl (meth)acrylate; and hydroxyl group-containing monomers such as hydroxyethyl (meth)acrylate and hydroxyethylpropyl (meth)acrylate. Additionally, a combination of one or two or more of monomers such as vinyl amines (e.g., vinyl pyridine, vinyl pyrrole, and vinyl imidazole), and acrylonitrile and styrene may also be used.

By copolymerizing these copolymerizable monomers within a range of 3 to 60% by weight to 40 to 97% by weight of the (meth)acrylic acid alkyl ester, preferably within a range of 5 to 40% by weight to 60 to 95% by weight of the (meth)acrylic acid alkyl ester, the adhesive with excellent adhesive properties for use in accordance with the present invention can be prepared.

Additives known as plasticizers, softening agents, fillers and tackifiers may appropriately be blended in the adhesive layer described above. In order to reduce the modulus of the adhesive layer in the low deformation region of the antimicrobial adhesive sheet of the present invention to improve skin adhesion more and in order to reduce cuticle damages and pains when the antimicrobial adhesive sheet is removed from the skin surface, fatty acid esters are preferably contained.

In order to avoid the occurrence of any deviation in the adhesive properties of the adhesive due to the inhibition of the adhesive properties thereof, preferably, the fatty acid esters contained have affinity and compatibility with the adhesive. Specifically, use can be made of carboxylic acid esters using monovalent alcohols, such as ethyl myristate, isopropyl myristate, isopropyl palmitate, butyl stearate, isopropyl isostearate, hexyl laurate, cetyl lactate, myristyl lactate, dietyl phthalate, octyldodecyl myristate, octyldodecyl oleate, hexyldecyl dimethyloctanoate, decyl 2-ethylhexanoate, isocetyl 2-ethylhexanoate, stearyl 2-ethylhexanoate, and dioctyl succinate; and carboxylic acid esters using polyvalent alcohols of divalence or more, such as propylene glycol dicaprylate, propylene glycol dicaprate, propylene glycol diisostearate, glyceryl monocaprylate, glyceryl tricaprylate, glyceryl tri(2-ethylhexanoate), glyceryl tricaprate, glyceryl trilaurate, glyceryl triisostearate, glyceryl trioleate, and trimethylol propane tri(2-ethylhexanoate).

Among these fatty acid esters, esters of saturated fatty acids with no unsaturated bond within each fatty acid molecule and glycerin are preferably used as a fatty acid ester with less oxidative deterioration from the standpoint of the stability of the quality. Particularly, triglycerol esters of caprylic acid, capric acid and 2-ethylhexanoic acid are preferably used.

One or two or more of the fatty acid esters may be used in combination as a component of the adhesive or may be used in combination to be blended with an acrylic copolymer as a component forming the adhesive. The amount thereof to be blended is 20 to 120 parts by weight to 100 parts by weight of the acrylic copolymer. Preferably, the amount is adjusted to a range of 30 to 100 parts by weight. When the amount of such fatty acid ester to be blended is too small, the resulting adhesive layer may not sufficiently absorb sweat from skin when the antimicrobial adhesive sheet of the present invention is attached on skin, so that the adhesiveness to skin may potentially be reduced. When the amount of such fatty acid ester to be blended is too high, the adhesive layer may be too much plasticized so that the adhesiveness to skin may sometimes be greatly reduced.

In case that the fatty acid ester is to be blended in the adhesive layer, the adhesive layer is preferably treated by crosslinking treatment so as to improve the retention of the fatty acid ester and make a balance between the adhesiveness of the adhesive layer to skin and the inner aggregation force. As the crosslinking treatment, chemical crosslinking treatment using intermolecular crosslinking agents of organic compounds such as organic peroxides, isocyanate compounds, epoxy compounds and metal chelate compounds and physical crosslinking treatment with irradiation of ionizing radiation are preferable so as to securely procure an appropriate free space in the polymers composing the adhesive in order to securely retain the fatty acid ester. For the chemical crosslinking treatment, specifically, organic peroxides such as benzoyl peroxide, isocyanate compounds and polyfunctional isocyanate compounds such as tolylene diisocyanate and hexamethylene diisocyanate, epoxy compounds such as glycerin triglycidyl ether and triglycidyl isocyanurate, metal chelate compounds such as aluminium tris(acetylacetonate) and ethyl acetoacetate aluminium diisopropylate are listed. Among them, isocyanate compounds, polyfunctional isocyanurate compounds and metal chelate compounds are preferably used from the standpoint of cross linking reactivity and easy handling.

For the physical crosslinking treatment, meanwhile, irradiation methods of electron beam, gamma ray and X ray are listed. From the safety and handleability standpoint, electron beam and gamma ray are preferably used. The irradiation dose is 20 to 50 kGy, preferably 25 to 35 kGy. By irradiation of ionizing radiation, further, not only the crosslinking treatment of the adhesive layer but also so-called sterilization treatment of the layer can be done. Thus, such irradiation is extremely appropriate for use in skin attachment in accordance with the present invention.

The vaporizable antimicrobial agent is contained in the non-aqueous adhesive layer in the antimicrobial adhesive sheet of the present invention. The vaporizable antimicrobial agent for potential use includes any antimicrobial agent with vaporizability together with antimicrobial activities such as sterilizing action and bacteriostat action, and specifically includes for example but is not limited to isothiocyanic acid esters, eucalyptus oil, menthol, rosemary, and hinokitiol, which are contained at an amount of about 0.01 to 10 % by weight in the non-aqueous adhesive layer. Among these vaporizable antimicrobial agents, extracts containing isothiocyanic acid esters as the main components as well as hinokitiol is preferably used in view of safety profile, wide antimicrobial spectrum, no ready occurrence of resistant bacteria, suppression of the growth of normal flora and spatial antimicrobial property. Particularly preferable is hinokitiol.

As the extracts containing isothiocyanic acid esters as the main components, extracts containing as the main components allyl isothiocyanate, phenyl isothiocyanate, methyl isothiocyanate, ethyl isothiocyanate, propyl isothiocyanate, isopropyl isothiocyanate, butyl isothiocyanate, isobutyl isothiocyanate, isoamyl isothiocyanate, benzyl isothiocyanate and cyclohexyl isothiocyanate as the bitter components of mustard and Japanese horseradish can be used.

As hinokitiol, additionally, naturally occurring hinokitiol extracted from essential oils derived from plants, preferably plants of Cupressaceae such as Hinoki cypress (especially, Taiwan Chamaecyparis obtuba, Thujopsis dolabrata var. hondai and Thujopsis dolabrata) or chemically synthesized hinokitiol may be satisfactory. Furthermore, hinokitiol for use in accordance with the present invention may be in the free form or a salt form. As such, inorganic salts such as metal salts for example sodium salt, potassium salt, magnesium salt, calcium salt, copper salt and zinc salt, and organic salts such as ethanolamine salt, diethanolamine salt, propanolamine salt, piperazine salt, piperidine salt, arginine salt, lysine salt and histidine salt may also be used.

Because some of such vaporizable antimicrobial agents as described above are poorly stable in terms of their contents since they vaporize during the production process or because they have a thermal decomposition property, preferably, they are included by inclusion compounds such as cyclodextrin and are then contained in the adhesive layer so as to improve the stability through the suppression of the vaporization, the prevention of the oxidation and photodecomposition, and the modification into sustainable property. As cyclodextrin, $\alpha$-cyclodextrin, $\beta$-cyclodextrin, and $\gamma$-cyclodextrin may be used. From the standpoint of the inclusion of a vaporizable antimicrobial agent, however, $\beta$-cyclo-dextrin is preferable. Further, cyclodextrin has a high safety profile in terms of food hygiene and can efficiently allow the antimicrobial activity of the vaporizable antimicrobial agent to be more effectively exerted in a more stable manner.

The antimicrobial adhesive sheet of the present invention is composed of the constitution described above. Until use, preferably, the surface of the non-aqueous adhesive layer is covered with a removable sheet and then, the whole sheet is packaged with an air-tight packaging material. On use, the removable sheet is peeled off and removed from the surface of the adhesive layer, to attach the adhesive layer on adherend substances such as skin. In such manner, the antimicrobial activity is exerted on a site in contact, while the vaporizable antimicrobial agent vaporizes from the back side of the support to turn the space in the vicinity of the attached site into an antimicrobial atmosphere to exert the excellent antimicrobial effect.

The antimicrobial adhesive sheet of the present invention is now specifically described in the following Examples. However, the invention is not limited to the Examples and may be applicable within a range not departing from the technical idea of the invention.

<Preparation of adhesive layer A>

A monomer mixture of 65 parts by weight of isononyl acrylate, 30 parts by weight of 2-methoxyethyl acrylate, and 5 parts by weight of acrylic acid was homogenously dissolved in and mixed with 80 parts by weight of toluene. By using 0.3 part by weight of azobisisobutyronitrile as a polymerization initiator, the copolymerization reaction was carried out, to obtain an acrylic copolymer.

33.3 parts by weight of a methanol solution containing an antimicrobial agent at 3 % by weight was mixed per 100 parts by weight of the resulting acrylic copolymer. After one side of a removable sheet was treated with a silicone resin for removable treatment, the resulting solution was coated on the removable treatment-processed side of the removable sheet, to a dry thickness of 30 $\mu$m. Then, the sheet was dried at 100 °C for 3 minutes, to prepare adhesive layer A containing the vaporizable antimicrobial agent.

<Preparation of adhesive layer B>

To 100 parts by weight of the acrylic copolymer obtained in the preparation of the adhesive layer A were blended 60 parts by weight of triglyceryl caprylate and 0.1 part by weight of a trifunctional isocyanate compound as a crosslinking agent (trade name of Coronate HL; manufactured by Nippon Polyurethane Industry Co., Ltd.) in toluene, to prepare a homogenous adhesive solution, into which the antimicrobial agent was mixed in the same manner as for the preparation of the adhesive layer A. In the same manner as in the preparation of the adhesive layer A, except for those described above, adhesive layer B containing the vaporizable antimicrobial agent was prepared.

Examples 1 through 9 and Comparative Example 1 through 4

The adhesive layer A or B prepared above was bonded on one side of the individual supports described in Table 1 by contact-bonding and transfer, to prepare the antimicrobial adhesive sheet of the present invention, which was sealed and packaged with a packaging material of a polyacrylonitrile/aluminium laminate structure, followed by irradiation of 25-kGy γ ray for sterilization.

Table 1

| Support type | |
|---|---|
| A | Polyether polyurethane (manufactured by Dainichiseika Color & Chemicals Mfg. Co., Ltd.; Resamine P-210; thickness: 30 $\mu$m) |
| B | Polyether polyamide (manufactured by Toray Industries, Inc.; PEBAX; thickness: 30 $\mu$m) |
| C | Polyester/pulp non-woven fabric (manufactured by Nippon Daishowa Paper Board; NS-3500; weight: 35 g/m$^2$; thickness: 100 $\mu$m) |
| D | Polytetrafluoroethylene-made porous membrane (manufactured by Nitto Denko Corporation; NTF1122; mean pore size: 0.2 $\mu$m; thickness: 80 $\mu$m) |
| E | Laminate of polyether polyurethane (manufactured by Dainichiseika Color & Chemicals Mfg. Co., Ltd.; Resamine P-210; thickness: 30 $\mu$m)/monoaxially stretched polypropylene, (manufactured by Gunze Limited; Silfan MTO; thickness: 40 $\mu$m) |
| F | Monoaxially stretched polypropylene (manufactured by Gunze Limited; Silfan MTO; thickness: 40 $\mu$m) |
| G | Polyethylene terephthalate (manufactured by Toray Industries, Inc.; W10; thickness: 12 $\mu$m) |
| H | Polyvinyl chloride (manufactured by Bando Chemical Industries, Ltd.; thickness: 90 $\mu$m) |

The antimicrobial adhesive sheets prepared in the individual Examples and Comparative Examples were tested by the following methods. The results are shown in Table 2.

<Moisture permeability>

The antimicrobial adhesive sheets prepared in the individual Examples and Comparative Examples were cut into a circle-shaped piece of 50 mm$\phi$, and were then attached downward on and adhered to the top opening of a cylinder-shaped glass container with an inner diameter of 40 mm and a height of 40 mm, which was preliminarily charged with 20 ml of distilled water, so that the side of the adhesive layer could face the inside of the glass container. The container was stored in a thermostat at constant moisture at 40°C and a relative humidity of 30%. 24 hours later, the change of the weight of the container was measured. When the weight of the container at the start of storage (time 0) was defined as $W_2$ and the weight of the container 24 hours later was defined as $W_3$, the moisture permeability was calculated by the following formula.

**Chemical formula 1:**

$$\text{Moisture permeability (g/m}^2 \cdot 24 \text{ hrs)} = (W_2 - W_3)/(0.02 \times 0.02 \times \pi)$$

<Vaporizability test (residual ratio of antimicrobial agent)>

The antimicrobial adhesive sheets prepared in the individual Examples and Comparative Examples were cut into a size of 30 mm x 30 mm, from which each removable sheet was peeled off and removed. Subsequently, the exposed side of each adhesive layer was attached on one side of a polyester film (Mitsubishi Chemical Corporation; Dia foil MRN75) to cover the side of the adhesive layer. This was stored in a glare-protected thermostat at 32°C. 7 days later, this was taken out to measure the amount of an antimicrobial agent remaining inside the antimicrobial adhesive sheet by high-performance chromatography. When the content on day 0 was defined as $W_0$ and the content after storage was defined as $W_1$, the residual ratio (%) was calculated by the following formula. The assay conditions for the antimicrobial agent (hinokitiol) by high-performance chromatography were as follows.
High-performance chromatographic apparatus : manufactured by Yokogawa Electric Corporation; Agilent 100 series
Column: manufactured by YMC; YMC-Pack Polymer C18 (4.6 mm$\phi$ × 15 cm) Column temperature: 30°C
Elution solvent: 10 mM-$KH_2PO_4$ + 0.1 % by weight of EDTA • 2Na (adjusted to pH 3.5, via $H_3PO_4$ addition)/acetonitrile = 65/35
Flow rate: 1.0 ml/min
UV wavelength for detection: 238 nm.

**Formula 2:**

$$\text{Residual ratio (\%)} = (W_1/W_0) \times 100$$

<Antimicrobial test>

*Staphylococcus aureus* was used as a bacterial species. The bacterial strain under storage was transplanted on an SCD agar culture medium (tryptosoya agar culture medium) for culturing at 37°C for 24 hours. 10 platinum loops of the grown bacteria were scraped with a platinum loop of an inner diameter of 1 mm, suspended in aseptic physiological saline and diluted to a bacterial solution for inoculation. A sensitive disk culture medium N-Nissui (manufactured by Nissui Pharmaceutical Co., Ltd.) was used as a culture medium, on which a bacterial amount of $10^6$ cfu/cm$^2$ was inoculated. Subsequently, an antimicrobial adhesive sheet cut in a size of 15 mm$\phi$ was arranged on the culture medium so that the side of the adhesive layer of the antimicrobial sheet could be in contact to the culture medium. After a lid was placed on the culture medium, the bacteria were cultured at 37°C for 24 hours, to measure the inhibitory zone after the completion of culturing to examine the antimicrobial activity.

Table 2

| | | Adhesive type | Support type | Moisture permeability of support (g/m$^2$ • 24 hrs) | Residual ratio of antimicrobial agent (one week later; %) | Antimicrobial activity from adhesive side (diameter in cm of inhibitory zone) | Antimicrobial agent |
|---|---|---|---|---|---|---|---|
| Examples | 1 | A | a | 1632 | 17.4 | 0 | hinokitiol |
| | 2 | A | a | 1632 | 100.0 | 24 | hinokitiol/ cyclodextrin inclusion body |
| | 3 | A | b | 1585 | 36.9 | 0 | hinokitiol |
| | 4 | A | c | 3271 | 28.3 | 0 | hinokitiol |
| | 5 | A | d | 3122 | 17.2 | 0 | hinokitiol |
| | 6 | A | e | 26 | 100.0 | 21 | hinokitiol |
| | 7 | A | e | 26 | 100.0 | 23 | hinokitiol/ cyclodextrin inclusion body |
| | 8 | A | e | 26 | 101.5 | 28 | hinokitiol |
| | 9 | B | e | 26 | 101.5 | 29 | hinokitiol |
| Comparative Examples | 1 | A | e | 26 | ---- | 0 | none |
| | 2 | A | f | 20 | 64.0 | 20 | hinokitiol |
| | 3 | A | g | 59 | 80.0 | 20 | hinokitiol |
| | 4 | A | h | 108 | 83.3 | 20 | hinokitiol |

As apparent in the results shown in Table 2, the antimicrobial adhesive sheets of the present invention exert excellent antimicrobial activities. Additionally the sheets in each entirety can have high moisture permeability because supports of high moisture permeability are used therein. Consequently, the antimicrobial agent contained therein vaporizes. Additionally, the antimicrobial adhesive sheets of Examples 6 through 9 are prepared by temporarily laminating the support of low moisture permeability as used in Comparative Example 2 at a removable state on the support of high moisture permeability as used in Example 1 and forming an adhesive layer on the film of the high moisture permeability, so the sheets in each entirety have low moisture permeability. Thus, it is indicated that the sheets have poor vaporizability of the antimicrobial agent as they are. In case of such sheets, the support of low moisture permeability is peeled off on a needed basis to make an antimicrobial atmosphere by allowing the antimicrobial agent to vaporize. Further, the results show that the antimicrobial adhesive sheets of Examples 1, 3 and 5 apparently have no antimicrobial activity. The results are caused by the use of the supports of high moisture permeability so that the vaporization of the antimicrobial agent from the back side of the supports is faster than the release of the antimicrobial agent from the side of the adhesive layers. As described below, it was confirmed that the antimicrobial atmosphere was generated. The antimicrobial activities were better than in the products of the other Examples.

As not shown in the Examples, an antimicrobial test was conducted by putting the side of the adhesive layer and/or the side of the support not directly contact to the culture medium but at a distance of 10 mm apart from the culture medium, so as to vaporize the antimicrobial agent. Consequently, an inhibitory zone with an excellent antimicrobial activity was formed in any of the products of the Examples.

**Claims**

1. An adhesive sheet for use in the antimicrobial treatment of a surface, which comprises a support and a non-aqueous adhesive layer provided on one side of the support, said adhesive layer comprising an adhesive and a vaporizable antimicrobial agent and the antimicrobial adhesive sheet having a moisture permeability of at least 1,000 g/m$^2$ • 24 hrs as a whole sheet, on an adherend allowing the antimicrobial adhesive sheet to exert the antimicrobial activity on the surface of the non-aqueous adhesive layer in contact to the adherend and simultaneously vaporizing the vaporizable antimicrobial agent from the back side of the support so as to make the space in the vicinity of the attached site into an antimicrobial atmosphere.

2. The adhesive sheet for use according to claim 1, wherein the support is a sheet comprising non-woven fabric or porous plastic.

3. The adhesive sheet for use according to claim 1, wherein the support is a non-porous plastic sheet.

4. The adhesive sheet for use according to claim 3, wherein the non-porous plastic sheet is a plastic sheet comprising a resin selected from the group consisting of polyurethane resins and polyamide resins.

5. The adhesive sheet for use according to claim 1, wherein the vaporizable antimicrobial agent is hinokitiol or an inclusion compound thereof.

6. The adhesive sheet for use according to claim 1, wherein the non-aqueous adhesive layer comprises an acrylic adhesive obtainable by polymerization of a (meth)acrylic acid alkyl ester as the main component.

7. The adhesive sheet for use according to claim 1 or 6, wherein the non-aqueous adhesive layer comprises a fatty acid ester.

8. The adhesive sheet for use according to claim 7, wherein the non-aqueous adhesive layer has been subjected to crosslinking treatment.

9. The adhesive sheet for use according to claim 1, wherein the moisture permeability of the whole sheet is within the range of from 1,000 to 4,000 g/m$^2$ • 24 hrs.

10. The adhesive sheet for use according to claim 1, wherein the antimicrobial adhesive sheet further comprises an underlining sheet having a moisture permeability of 200 g/m$^2$ • 24 hrs or less which is laminated in a removable manner on the side of the support opposite to the side of the non-aqueous adhesive layer.


**Patentansprüche**

1. Klebefolie zur Verwendung bei der antimikrobiellen Behandlung einer Oberfläche, welche einen Träger und eine nichtwässrige Klebstoffschicht, die auf einer Seite des Trägers bereitgestellt ist, umfasst, wobei die Klebstoffschicht einen Klebstoff und einen verdampfbaren antimikrobiellen Wirkstoff umfasst und die antimikrobielle Klebefolie eine Feuchtigkeitsdurchlässigkeit von wenigstens 1000 g/m$^2$ • 24 h als eine ganze Folie aufweist, an einem Adhärenden, wobei es ermöglicht wird, dass die antimikrobielle Klebefolie die antimikrobielle Wirkung auf der Oberfläche der nichtwässrigen Klebstoffschicht in Kontakt mit dem Adhärenden ausübt, und gleichzeitig der verdampfbare antimikrobielle Wirkstoff von der Rückseite des Trägers verdampft wird, so dass in dem Raum in der Umgebung der Stelle, an der die Klebefolie angebracht ist, eine antimikrobielle Atmosphäre erzeugt wird.

2. Klebefolie zur Verwendung nach Anspruch 1, wobei der Träger eine Folie ist, die ein Faservlies oder einen porösen Kunststoff umfasst.

3. Klebefolie zur Verwendung nach Anspruch 1, wobei der Träger eine nicht-poröse Kunststofffolie ist.

4. Klebefolie zur Verwendung nach Anspruch 3, wobei die nicht-poröse Kunststofffolie eine Kunststofffolie ist, die ein Harz umfasst, das ausgewählt ist aus der Gruppe bestehend aus Polyurethanharzen und Polyamidharzen.

5. Klebefolie zur Verwendung nach Anspruch 1, wobei der verdampfbare antimikrobielle Wirkstoff Hinokitiol oder eine

10

Einschlussverbindung davon ist.

6. Klebefolie zur Verwendung nach Anspruch 1, wobei die nichtwässrige Klebstoffschicht einen Acrylklebstoff umfasst, der durch Polymerisation eines (Meth)acrylsäurealkylesters als die Hauptkomponente erhältlich ist.

7. Klebefolie zur Verwendung nach Anspruch 1 oder 6, wobei die nichtwässrige Klebstoffschicht einen Fettsäureester umfasst.

8. Klebefolie zur Verwendung nach Anspruch 7, wobei die nichtwässrige Klebstoffschicht einer Vernetzungsbehandlung unterzogen worden ist.

9. Klebefolie zur Verwendung nach Anspruch 1, wobei die Feuchtigkeitsdurchlässigkeit der ganzen Folie im Bereich von 1000 bis 4000 g/m$^2$ • 24 h liegt.

10. Klebefolie zur Verwendung nach Anspruch 1, wobei die antimikrobielle Klebefolie außerdem eine darunterliegende Lage mit einer Feuchtigkeitsdurchlässigkeit von 200 g/m$^2$ • 24 h oder weniger umfasst, welche auf eine entfernbare Weise auf die Seite des Trägers laminiert ist, die der Seite der nichtwässrigen Klebstoffschicht gegenüberliegt.

**Revendications**

1. Feuille adhésive à utiliser dans le traitement antimicrobien d'une surface, qui comprend un support et une couche adhésive non aqueuse prévue sur une face du support, ladite couche adhésive comprenant un adhésif et un agent antimicrobien vaporisable, et la feuille adhésive antimicrobienne dans son ensemble présentant une perméabilité à l'humidité d'au moins 1.000 g/m$^2$.24 heures, sur une surface de fixation permettant à la feuille adhésive antimicrobienne d'exercer l'activité antimicrobienne à la surface de la couche adhésive non aqueuse en contact avec la surface de fixation et de vaporiser simultanément l'agent antimicrobien à partir du dos du support de façon à placer dans une atmosphère antimicrobienne l'espace au voisinage du site de fixation.

2. Feuille adhésive à utiliser selon la revendication 1, dans laquelle le support est une feuille comprenant un tissu non tissé ou un plastique poreux.

3. Feuille adhésive à utiliser selon la revendication 1, dans laquelle le support est une feuille en plastique non poreux.

4. Feuille adhésive à utiliser selon la revendication 3, dans laquelle la feuille en plastique non poreux est une feuille en plastique comprenant une résine choisie dans le groupe formé de résines de polyuréthane et de résines de polyamide.

5. Feuille adhésive à utiliser selon la revendication 1, dans laquelle l'agent antimicrobien vaporisable est l'hinokitiol ou un composé d'inclusion de celui-ci.

6. Feuille adhésive à utiliser selon la revendication 1, dans laquelle la feuille adhésive non aqueuse comprend un adhésif acrylique obtenu par polymérisation d'un ester d'acide (méth)acrylique comme composant principal.

7. Feuille adhésive à utiliser selon la revendication 1 ou 6, dans laquelle la couche adhésive non aqueuse comprend un ester d'acide gras.

8. Feuille adhésive à utiliser selon la revendication 7, dans laquelle la couche adhésive non aqueuse a subi un traitement de réticulation.

9. Feuille adhésive à utiliser selon la revendication 1, dans laquelle la perméabilité à l'humidité de l'ensemble de la feuille se situe dans l'intervalle de 1.000 à 4.000 g/m$^2$.24 heures.

10. Feuille adhésive à utiliser selon la revendication 1, dans laquelle la feuille adhésive antimicrobienne comprend de plus une feuille de doublure présentant une perméabilité à l'humidité de 200 g/m$^2$.24 heures ou moins, qui est lamifiée de manière amovible sur la face du support opposée à la face de la couche adhésive non aqueuse.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0074737 A
- JP 61002862 A
- JP 2002272831 A